(19) Europäisches Patentamt
European Patent Office
Office européen des brevets

(11) **EP 4 446 855 A1**

(12) **EUROPEAN PATENT APPLICATION**

(43) Date of publication:
**16.10.2024 Bulletin 2024/42**

(21) Application number: **23305551.6**

(22) Date of filing: **12.04.2023**

(51) International Patent Classification (IPC):
*G06F 3/0346* (2013.01)

(52) Cooperative Patent Classification (CPC):
**G06F 3/0346; G01D 5/145; G01R 33/0094;
G01R 33/072; G01R 33/10; G06F 3/033;
G06F 3/0338;** A61B 34/30; G05G 2009/04755

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **MinMaxMedical
38400 Saint-Martin-d'Hères (FR)**

(72) Inventors:
• **SEDNAOUI, Thomas
38400 SAINT-MARTIN-D'HÈRES (FR)**
• **ODET, Baptiste
38400 SAINT-MARTIN-D'HÈRES (FR)**

(74) Representative: **Regimbeau
20, rue de Chazelles
75847 Paris Cedex 17 (FR)**

(54) **METHOD FOR TRACKING A MAGNETIC FIELD SOURCE OF AN INPUT DEVICE**

(57)     A method for determining a position of a movable part of an input device relative to a static part of the input device comprises measuring a magnetic field generated by a magnetic source with a sensor array. The magnetic source is coupled to one of the movable part and the static part and the sensor array is coupled to the other of the movable part and the static part. The sensor array comprises a plurality of unidirectional sensors that measure a portion of the magnetic field parallel to a direction of magnetization of the magnetic source. The movable part is displaceable relative to the static part according to at least two and at most three degrees of freedom. The degrees of freedom being two degrees of translational displacement and one degree of rotational freedom. The method further comprises determining a location within a coordinate system of the sensor array at which the magnitude of the magnetic field of the magnetic source is the greatest.

*FIGURE 9*

**Description**

TECHNICAL FIELD

The present disclosure relates generally to a tracking system for an input device. The input device is adapted to guide movement of a component of a system with which the input device is operatively connected. More particularly, the present disclosure is related to a method and system for tracking movement of the input device.

BACKGROUND

Systems having magnetic sources and magnetic sensors for tracking movement of input devices are described in, for example, US8862200 and WO2022018691.

The computer-assisted surgery systems of US886220 and WO2022018691 utilizes a magnetic source to locate a position of a bone of a patient to which the magnetic source is coupled. WO2022018691 further describes using the magnetic source to track displacements of a surgical tool in a patient's body. As the magnetic sources have six degrees of freedom, the systems must employ magnetic sensors that measure the magnitude of all components of the three-dimensional magnetic flux density of the magnetic field of the magnetic sources.

BRIEF SUMMARY

The input device and associated method of the present disclosure may utilize a unidirectional sensor to measure a single directional component of a magnetic field emitted by a magnetic sources. By using a unidirectional sensor, the tracking method may be simplified by reducing the quantity of magnetic field components to be measured and analyzed in order to locate and track the magnetic source without relying on the magnetic source field model. Such tracking method may have the further advantage of increased processing speed without reducing precision of following changes in the position of a movable part of the input device over time.

The input device and associated method of the present disclosure may utilize an input device having at least two degrees of freedom and, preferably, may have only three degrees of freedom. Such an input device may be simpler and less expensive to manufacture relative to input devices having more degrees.

A method for determining a position of a movable part of an input device relative to a static part of the input device comprises measuring a magnetic field generated by a magnetic source with a sensor array. The magnetic source is coupled to one of the movable part and the static part and the sensor array is coupled to the other of the movable part and the static part. The sensor array comprises a plurality of unidirectional sensors that measure a portion of the magnetic field parallel to a direction of magnetization of the magnetic source. The movable part is displaceable relative to the static part according to at least two and at most three degrees of freedom. The degrees of freedom being two degrees of translational freedom and one degree of rotational freedom. The method further comprises determining a location within a coordinate system of the sensor array at which the magnitude of the magnetic field of the magnetic source is the greatest.

Certain preferred but non-limiting features of the method described above are the following, taken individually or in combination:

    determining a displacement of the magnetic source based on the location at which the magnitude of the magnetic field of the magnetic source is the greatest relative to an initial position of the magnetic source within the coordinate system of the sensor array;
    determining a location at which the magnitude of the magnetic field of the magnetic source is the greatest comprises: determining a surface fit to the plurality of magnetic field measurements of the sensors and determining an apex of the surface and a location of the apex within the coordinate system of the sensor array;
    determining a location at which the magnitude of the magnetic field of the magnetic source is the greatest comprises computing a centroid of the plurality of discrete magnetic field measurements of the sensors
    determining the displacement of the magnetic source comprises determining at least one of translational displacement and angular displacement of the magnetic source relative to the initial position;
    measuring the magnetic field generated by the magnetic source with the sensor array comprises: measuring a magnetic field generated by a first magnetic source with a first portion of the sensor array and measuring a magnetic field generated by a second magnetic source with a second portion of the sensor array, wherein the first magnetic source and the second magnetic source are coupled to the one of the movable part and the static part such that the first and second magnetic sources are separated by a fixed distance;
    determining the displacement of the magnetic source relative to the initial position of the magnetic source comprises determining a rotational displacement of the first magnetic source and the second magnetic source;

displacing a part of a system to which the input device is operatively connected based upon the determined displacement of the magnetic source;

prior to determining the location at which the magnitude of the magnetic field of the magnetic source is the greatest, detecting an outlier magnetic field measurement from the measured magnetic field of the sensors of the sensor array;

removing the outlier magnetic field measurement and using remaining magnetic field measurements to determine the location at which the magnitude of the magnetic field of the magnetic source is the greatest; and/or

communicating an error to a user based on the outlier magnetic field measurements.

A system for tracking displacement of a movable part of an input device relative to a static part of the input device comprises at least one magnetic source coupled to one of the movable part and the static part. The movable part exhibits three degrees of freedom, and the three degrees of freedom consist of two degrees of translational freedom and one degree of rotational freedom. The input device further comprises an array of sensors coupled to the other of the movable part and the static part and a control unit configured to perform the method as previously described;

Certain preferred but non-limiting features of the device described above are the following, taken individually or in combination:

the sensors are Hall effect sensors;
the at least one magnetic source is a permanent magnet; and/or
the at least one magnetic source is coupled to the one of the movable part and the static part such that a direction of magnetization of the at least one magnetic source is parallel to an axis of rotational freedom of the input device.

A computer-assisted surgical system comprises a robotic arm with a plurality of motorized joints, a surgical tool coupled to the robotic arm, and the system as previously described, wherein the control unit is configured to displace at least one of the robotic arm and the surgical tool based on the determined displacement of the magnetic source.

BRIEF DESCRIPTION OF THE DRAWINGS

The illustrations presented herein are not meant to be actual views of any particular component, device, or system, but are merely idealized representations which are employed to describe embodiments of the present invention. Other features, goals, and advantages of the present invention will appear more clearly upon reading of the detailed description that follows and with references to drawings provided by way of non-limiting examples wherein:

FIGS. 1 and 2 illustrate a part of an input device including magnetic sources and sensors;
FIGS. 3-5 illustrate relative placement of the sensors and magnetic sources of the input device;
FIGS. 6 and 7 are exemplary plots of the measure magnetic field intensity as a function of location of the sensors;
FIGS. 8a and 8b illustrate positions of the magnetic sources in order to determine displacement of the magnetic sources according to methods of the present disclosure;
FIG. 9 illustrates a computer-assisted surgery (CAS) system including the input device of the present disclosure; and
FIG. 10 illustrates a schematic diagram of the general architecture of a system for use in the method of the present disclosure.

DETAILED DESCRIPTION

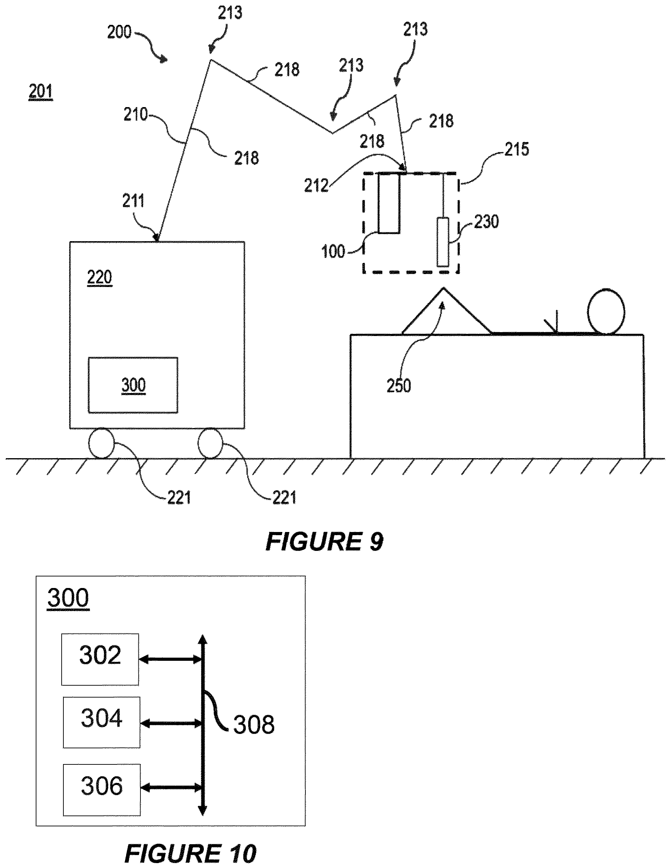

As used herein, the term "and/or" includes any and all combinations of one or more of the associated listed items.
As used herein, the term "configured" refers to a size, shape, material composition, material distribution, orientation, and arrangement of one or more of at least one structure and at least one apparatus facilitating operation of one or more of the structure and the apparatus in a pre-determined way.
As used herein, the term "part" as used in "static part" and "movable part" herein are both singular and plural. For instance, a static part of an input device as described herein may be formed by a single component or by multiple components or pieces assembled together in a manner that the component(s) or pieces are fixed relative to the structure to which the input device is coupled.
The present application relates to an input device. The input device 100 is a piece of equipment, such as a joystick or human interface device, that can be manipulated by a user to provide control signals to a system to which the input device 100 is operatively coupled. By way of example, the input device 100 may be part of a computer-assisted surgery (CAS) system 200 described with reference to FIG. 9 herein. The input device 100 may be configured to be grasped by a user, such as a surgeon, and moved by the user. Movement of the input device 100 is intended to result in movement of a robotic arm and/or a surgical tool in a surgical procedure. The present disclosure is not limited to use of the input device 100 in a CAS system. By way of further example, the input device 100 may be used in gaming device controllers, in controllers for machinery like cranes, trucks, unmanned vehicles, wheelchairs, and other domains that would benefit from an accurate input device with a long lifespan, reliability, error detection, and safety as described herein.

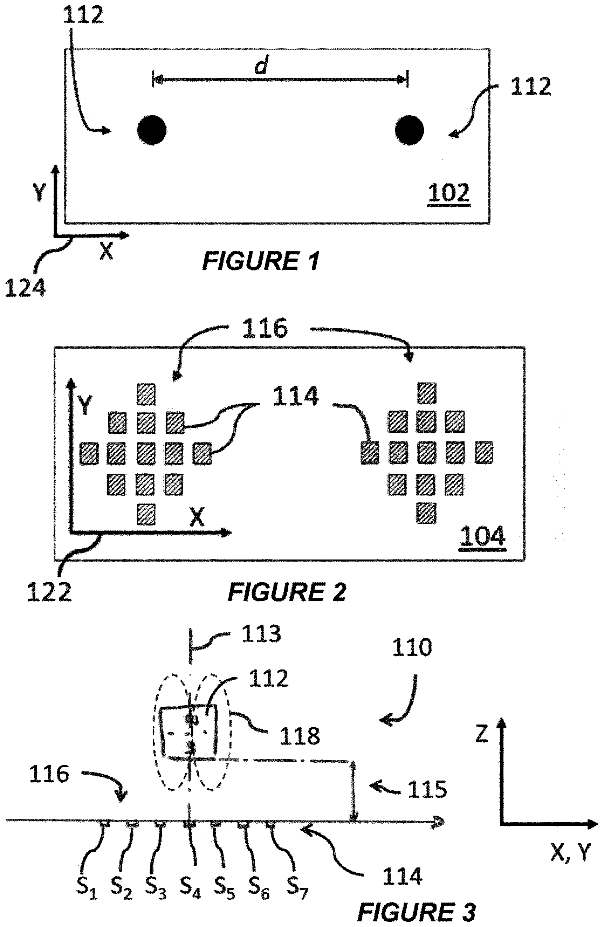

The input device 100 comprises a movable part 102 (FIG. 1) and a static part 104 (FIG. 2). The static part 104 is static relative to the device or system to which the input device 100 is coupled, and the movable part 102 is movable relative to the static part 104. For example, the static part 104 may be fixedly coupled to a segment 218 of a robotic arm 210 of a surgical system such that the static part 104 is static (e.g., not movable) relative to the segment 218 of the robotic arm 210 to which the part 104 is coupled, and the movable part 102 may be coupled to the static part 104 such that the movable part 102 is movable (e.g., displaceable) relative to the static part 104 and relative to the segment 218 of the robotic arm 210.

The movable part 102 is configured to exhibit at least two and up to three degrees of freedom. The three degrees of freedom consist of two degrees of translational freedom and one degree of rotational freedom. The movable part 102 is translatable forward-backward along the x-axis, translatable side-to-side along the y-axis, and rotatable (e.g., yaw) about the z-axis (e.g., normal axis). These axes are illustrated in FIG. 3. The movable part 102 and the static part 104 are separated by a gap 115 along the z-axis. This arrangement of the static part 104 and the movable part 102 prevents wear and friction generation at an interface therebetween so as to extend the functional life of the input device 100.

The input device 100 comprises a system 110 for tracking movement of the movable part 102 relative to the static part 104. The system 110 is a contactless system for determining displacement of the movable part 102 relative to the static part 104.

The system 110 includes at least one magnetic source 112 configured to emit a magnetic field (schematically illustrated by lines 118) and a plurality of sensors 114 arranged in one or more arrays 116 configured to measure the magnetic field of the magnetic source 112.

In the illustrated embodiments, the system 110 may include two magnetic sources 112.

In some embodiments, the system 110 may include three or more magnetic sources 112 arranged in a non-symmetrical pattern. Such a pattern provides a method to detect 180-degree rotation of the movable part 102, which be undetectable with only two magnetic sources 112 symmetrically arranged on the movable part 102.

The magnetic source 112 may be a permanent dipole magnet or a temporary magnet. The magnetic source may have a cylindrical shape, which is circular in cross-sectional shape as illustrated in FIG. 1, though the person skilled in the art would understand magnets of other shapes could be used, and/or a combination of magnet and magnetic guide.

The magnetic source 112 may be coupled to one of the movable part 102 and the static part 104, and the sensors 114 are coupled to the other of the movable part 102 and the static part 104. For simplicity of description, the system 110 will only be described with respect to an embodiment in which the magnetic source 112 is coupled to the movable part 102 and the sensors 114 are coupled to the static part 104.

The magnetic source 112 is coupled to the movable part 102 such that a direction of magnetization indicated by axis 113 extends substantially perpendicular (e.g., normal) to a translation plane in which lies the x-axis and y-axis along which the movable part 102 is translatable.

The magnetic sources 112 are fixed to the movable part 102 such that the magnetic sources 112 are separated by a fixed distance $d$. The fixed distance $d$ may be selected such that the magnetic fields of the respective magnetic sources 112 do not substantially interfere with each other. In some embodiments, the magnetic sources 112 may be coupled to a common substrate or may be coupled to separate substrates otherwise coupled together. The fixed distance $d$ is a parameter known and stored in a memory 304 of a control unit 300 (FIG. 10). The dimensions of the magnetic source 112 including, but not limited to, the radius of the magnetic source may also be known and is a parameter that may optionally be stored in the memory 304 of the control unit 300.

With reference to FIG. 2, the sensors 114 are fixed to the static part 104. In some embodiments, the sensors 114 may be fixed to a common surface of a substrate of the static part 104. In other embodiments, a first plurality of sensors 114 may be coupled to a first side of the substrate and a second plurality of the sensors 114 may be coupled to an opposing, second side of the substrate. The distance in the z-direction of each sensor 114 from the magnetic source 112 is known and is a parameter stored in the memory 304 of the control unit 300.

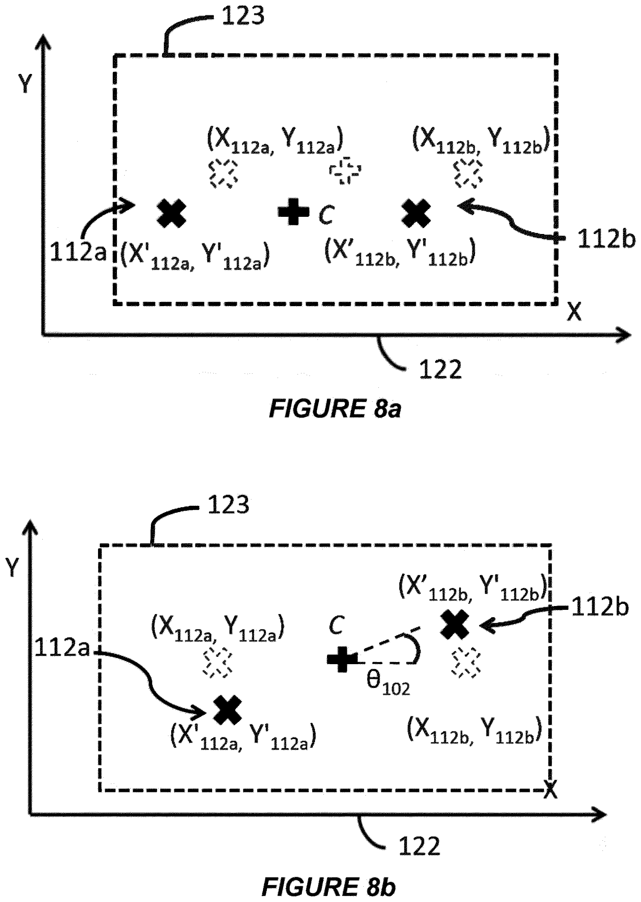

In the illustrated embodiments, the sensors 114 are arranged in a plane 123 (FIG. 8) extending in the x-direction and y-direction. A location (i.e., X and Y coordinates) of each sensor 114 within a coordinate system 122 of the array 116 are known and are parameters stored in the memory 304 of the control unit 300. The sensors 114 are arranged such that, in operation, each magnetic source 112 will be detected by at least two sensors 114 of the array 116.

The x-axis and y-axis of the coordinate system 122 of the sensor array 116 extend parallel to the x-axis and y-axis, respectively, of the coordinate system 124 of the movable part 102. The x-axes and the y-axes of each of the coordinate systems 112, 124 are perpendicular to the direction of magnetization 113 of the magnetic source 112.

The sensors 114 are separated by a known distance, or pitch, and arranged at known locations (i.e., x and y coordinates). The pitch and locations of the sensors 114 are fixed such that the sensors 114 of a given array 116 are not movable relative to each other. The locations or pitch of the sensors 114 is known and stored in the memory 304 of the control unit 300.

The pitch of the sensors 114 within a given array 116 may be variable. For example, the distance between adjacent sensors 114 may increase as the distance from the geometric center of the array 116 increases. Alternatively, the pitch of the sensors 114 may be constant. In such an embodiment, the distance between adjacent sensors 114 does not change as the distance from the geometric center of the array 116 increases.

Each magnetic source 112 is associated with a portion of the sensors 114 of the sensor array 116. The array 116 includes at least three sensors 114 for each magnetic source 112 included in the device 100. The number of sensors 114 in the input device 100 is selected based on the desired measurement precision needed and range of motion for the particular application of the input device 100. In addition, increasing the number of sensors enables redundancy.

The sensors 114 may be Hall effect sensors. Hall effect sensors measure the intensity (i.e., strength) of the magnetic field of the magnetic source 112 and output a voltage that is proportional to that magnetic field measurement, at least in a given magnetic field range.

The sensors 114 may be unidirectional sensors. Unidirectional sensors are configured to measure the magnetic field emitted by the magnetic source 112 in a single direction. In particular, the sensors 114 are configured to measure an intensity of the normal component of the magnetic field, which is the portion of the magnetic field parallel to the direction of magnetization (i.e., parallel to axis 113) and perpendicular to the x-axis and y-axis of the coordinate system of the sensor array 116. Each sensor 114 detects and measures a local magnetic field intensity at the respective positions.

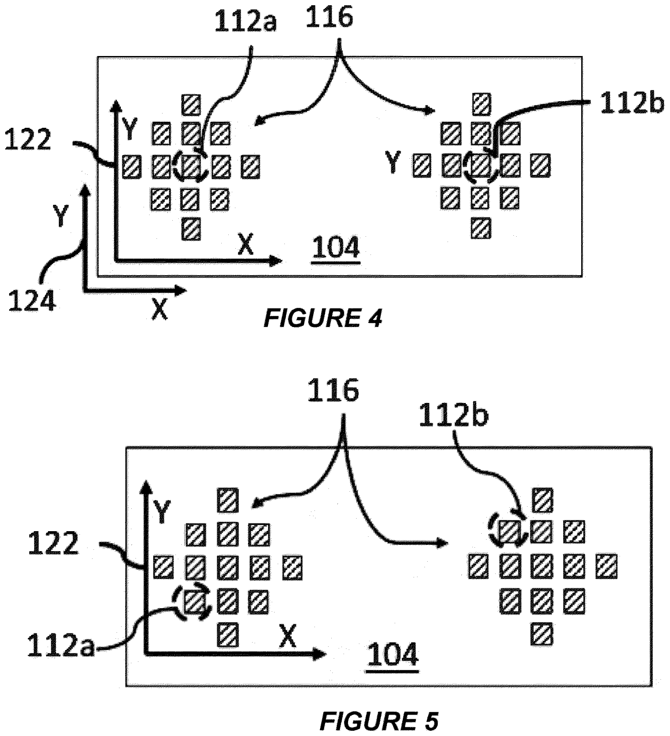

When the system 110 is assembled, the magnetic source 112 is disposed over the array 116 of the sensors 114. This arrangement is illustrated in FIGS. 4 and 5 wherein the magnetic sources 112 are illustrated by dashed lines. This arrangement is also illustrated in the cross-sectional view of FIG. 3.

FIG. 3 illustrates the system 110 in a neutral position. The neutral position refers to the arrangement of the movable part 102 relative to the static part 104 when the movable part 102 is not acted upon by a user. The input device 100 may include an elastic return means that urges the movable part 102 to the neutral position. The elastic return means may be a spring, an elastic band, a magnetic device, pneumatic device, and the like.

One of the sensors 114 of a given portion of the array 116 associated with an individual magnetic source 112 may be referred to herein as a reference sensor. The reference sensor is the sensor located closest to the magnetic source 112 and will have the highest measured magnetic field strength relative to the remaining sensors of the array when the movable part 102 and the static part 104 are in the neutral position. As the movable part 102 is moved in at least one of the x-direction and y-direction away from the neutral position, the strength of the measured magnetic field of the reference sensor may change.

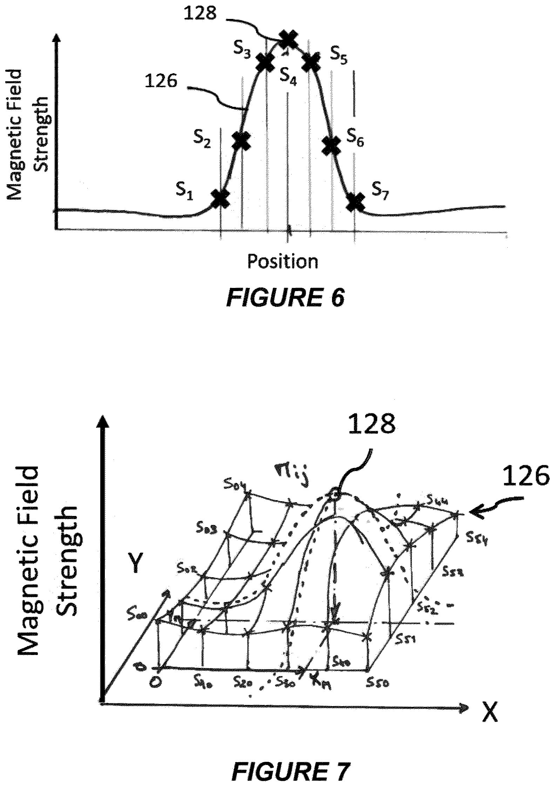

FIG. 6 illustrates a plot of the measurement of the axial magnetic field strength by each sensor 114 (y-axis) as a function of discrete location of the sensors along the x-direction and a curve (e.g., taking into consideration the location of the sensor along only one of the x-direction and y-direction) fit to the measurements. FIG. 7 illustrates a plot of the measurement of the magnetic field by each sensor 114 in the x-direction and the y-direction and a surface 126. The magnitude of the measured magnetic field of each sensor ($S_1$-$S_7$) is illustrated in FIG. 6 and 7. FIGS. 6 and 7 further illustrate a surface 126 fitted to the sensor measurements.

In FIG. 6, the sensor $S_4$ having the greatest magnetic field measurement is located closest to magnetic source 112 and serves as a reference sensor. When the movable part 102 is in a neutral position, an apex 128 of a surface 126 fitted to the measured magnetic field of each sensor is located coincident with the reference sensor $S_4$.

In operation, the system 110 is used to determine a position of the magnetic source(s) 112 relative to the array 116 of sensors 114. More particularly, displacement of the magnetic source(s) 112 relative to an initial position may be determined. The position and/or displacement of the magnetic source(s) 112 may be used to determine position and/or displacement of the movable part 102 relative to the static part 104.

The initial position may be the position of the magnetic source(s) 112 relative to the array 116 when the movable part 102 and the static part 104 are in the neutral position.

The method includes measuring a magnetic field generated by the magnetic source 112 with the sensors 114 of the sensor array 116. Each sensor 114 of the array 116 measures a local portion of the magnetic field of the magnetic source 112.

When the sensors 114 are Halls effect sensors, measuring the magnetic field of the magnetic source 112 comprises measuring a voltage representative of the magnitude of the magnetic field at a given sensor 114. Further, when the sensors 114 are unidirectional sensors, measuring the magnetic field of the magnetic source 112 comprises measuring a normal component of the magnetic field of the magnetic source 112, which normal component is parallel to the direction of magnetization of the magnetic source 112.

The magnetic field intensity measurement for each sensor 114 is received at the control unit 300. As understood by the person skilled in the art, the measurements may be normalized, linearized, corrected from offset, or otherwise modified depending upon the magnetic sensor technology used for the sensors 114.

The method further includes determining, at the control unit 300, a position (e.g., location) of the magnetic source(s) 112 within the coordinate system 122 of the sensor array 116.

The position of the magnetic source 112 may be located by determining a location within the coordinate system

122 of the sensor array 116 at which the magnitude of the magnetic field of the measured magnetic source 112 is the greatest.

In some embodiments, the method includes fitting a curved surface 126 to a plurality of discrete measured magnetic field outputs from the sensors 114 and determining a position of an apex 128 of the surface 126. The apex 128 of the surface 126 being the location at which the magnetic field of the measured magnetic source 112 is the greatest.

The surface fitting method may be any appropriate surface fitting such as a polynomial surface fitting, a digital surface fitting, or an electronically analogous surface fitting. The position of the magnetic source 112 may also be located by computing a centroid of the plurality of discrete magnetic field measurements from the sensors 114 in analogous or digital mode.

As the apex 128 of the fitted surface 126 may be located between two or more sensors 114, the apex 128 may be interpolated from the fitted surface 126. The apex 128 is indicative of the location of the magnetic source 112 relative to the array 116 of the sensors 114.

Alternatively or additionally, the position of the magnetic source 112 may be located by comparing the measured magnetic field outputs from the sensors 114 to a previously registered to a pre-registered pattern of magnetic field outputs that would be expected magnetic field outputs for a given arrangement of magnet sources 112 and sensors 114 spaced apart at a given distance from each other.

The step of the determining the location of the apex 128 is repeated for each magnetic source 112 and associate portion of the sensor array 116.

As the magnetic source 112 is coupled to the movable part 102 of the input device 100, the location of the apex 128 is indicative of the location of the movable part 102 relative to the static part 104. The method further comprises determining the location of the apex 128 within the coordinate system 124 of the movable part 102 such as by transforming the location of the apex 128 from the coordinate system 122 of the sensor array 116 to the coordinate system 124 of the movable part 102 of the input device 100.

Optionally, the method may also comprise detecting outlier measurements from the discrete sensor measurements. Detecting outlier measurements may include a regression analysis of the sensor measurements and computing a statistical likelihood that any given sensor output measurement is incoherent with neighboring sensor measurements, and/or that the surface fitting is more statistically significant if such sensor measurement is eliminated. Such analysis may be based upon an expected surface fit for a given magnetic source 112 and sensor array 116 determined at the time of system 110 manufacturing.

The number of outliers and the relative positioning of the outliers may be indicative of an error or other malfunction of the system 110, the input device 100, or a component thereof. By way of example, where two closely located sensors 114 are repeatedly determined to be outliers, this indicates that such sensors 114 are faulty and, thus, inhibit accurate determinations of the magnetic source 112 especially when the magnetic source 112 is located close to such faulty sensors. Given the high accuracy required for certain applications of the system 110 and input device 100, especially in the medical field in which accurate tool positioning and movement provided by a CAS system reduces the risk of injury to patient tissue adjacent an area of operation, this additional step of identifying outliers may increase overall system safety and accuracy of the medical procedure. The method may, therefore, comprise communicating the error via the control unit 300 to a user when the error is detected based upon the outlier sensor measurements.

The method may optionally comprise a step of eliminating outlier measurements from the discrete sensor measurements. Eliminating outlier measurements may result in a change to the surface 126 fitted to the discrete sensor measurements such that the apex 128 is more accurately located. This additional step of eliminating outliers may increase overall system safety and accuracy of the medical procedure.

The method may optionally comprise a step of normalizing the magnetic field measurements. Alternatively or additionally, prior to fitting the surface 126 to the discrete sensor measurements, the method may optionally comprise a step of correcting the magnetic field measurements based on sensitivities of the sensors, such as temperature sensitivity.

Optionally, in addition to determining the location of the apex 128, the method may also comprise determining the magnetic field strength of the apex 128. When the input device 100 is manufactured, the maximum magnetic field strength measurable by the sensors 114 for a given magnetic source 112 may be known parameter and may be stored in the memory 304 of the control unit 300. The method may include a step of comparing the maximum magnetic field strength determined from the sensor measurements to the known maximum magnetic field strength. When the difference between the determined and known maximum magnetic field strength exceeds a pre-determined threshold, this may be an indication that the input device 100, the system 110, or one or more components thereof is not properly functioning and may require repair or replacement. Thus, given the high accuracy required for certain applications of the system 110 and input device 100, especially in the medical field as previously explained, this additional method step increases overall system safety and accuracy of the medical procedure.

The method further includes determining a displacement of the magnetic source 112 relative to the initial position. The location of the magnetic source 112 is known based on the initial manufacture of the input device 100.

Thus, the displacement of the magnetic source 112 relative to the initial position may be determined by comparing the coordinates of the magnetic source 112 determined from the apex 128 and the coordinates of the magnetic source 112 in the initial position.

Determining the displacement of the magnetic source 112 includes determining at least one of the translational displacement of the magnetic source 112 (i.e., the displacement of the magnetic source in at least one of the x-direction and the y-direction) and angular displacement.

For purposes of explanation of methods of the present disclosure, FIG. 8a demonstrates a potential relative position of magnetic sources 112a, b when the movable part 102 is displaced in a purely translational manner and FIG. 8b demonstrates a potential relative position of magnetic sources 112a, 112b when the movable part 102 is angularly displaced. The original placement of the magnetic sources 112a, 112b is indicated by a X in dashed lines, while the displaced placement of the magnetic sources 112a, 112b is indicated by an X in solid lines.

With reference to FIGS. 8a and 8b, the initial coordinates of the magnetic sources 112a, 112b are known and identified in FIGS. 8a and 8b as $(X_{112a}, Y_{112a})$ and $(X_{112b}, Y_{112b})$, respectively, within the coordinate system 122 of the array 116. The coordinates of the magnetic source 112a, 112b after being displaced are determined and identified as $(X'_{112a}, Y'_{112a})$ and $(X'_{112b}, Y'_{112b})$, respectively, within the coordinate system 122 of the array 116.

When the displacement of the movable part 102 relative to the static part 104 is only translational, the displacement may be determined based upon the displacement of one of the magnetic sources 112 relative to the array 116 of sensors 114. The translational displacement in the x-direction $Tx_{102}$ and in the y-direction $Ty_{102}$ of the movable part 102 relative to the static part 104 may be determined as set forth below depending on whether the translation is in the x-direction (Equation 1) and/or y-direction (Equation 2):

$$Tx_{102} = X'_{112a} - X_{112a} = X'_{112b} - X_{112b} \quad (1)$$

or

$$Ty_{102} = Y'_{112a} - Y_{112a} = Y'_{112b} - Y_{112b} \quad (2)$$

When the movable part 102 is angularly displaced, the displacement may be determined as an angle $\theta_{102}$ in which d is the distance between magnetic sources 112, as set forth in Equation 3:

$$\theta_{102} = \arcsin\left(\frac{Y'_{112a} - Y'_{112b}}{d}\right) - \arcsin\left(\frac{Y_{112a} - Y_{112b}}{d}\right) \quad (3)$$

While described separately herein, the method may include determining both angular displacement and translational displacement simultaneously. Translational displacement and angular displacement may be determined by searching for the displacement of a point C centrally located between the magnetic sources 112a, 112b using the Equation (4) to determine x,y translations of the point C:

$$\left(\frac{(X'_{112a} + X'_{112b}) - (X_{112a} + X_{112b})}{2}\right), \left(\frac{(Y'_{112a} + Y'_{112b}) - (Y_{112a} + Y_{112b})}{2}\right) \quad (4)$$

The method further comprises transmitting the determined translational and/or rotational displacement of the magnetic sources 112 to the system to which the input device 100 is operatively coupled. As a result, one or more components of the system to which the input device 100 is coupled may be displaced in a manner corresponding to the translational and/or rotational displacement of the movable part 102 relative to the static part 104.

The foregoing method may be repeated for continuous tracking of the movement of the movable part 102 over time. In such embodiments, the initial position used to determine the displacement of the movable part 102 relative to the static part 104 may be a previously determined displacement of the movable part 102.

The method may also include monitoring of a distance between the magnetic sources 112a, 112b based upon the determined position thereof. The distance between the magnetic sources 112a, 112b may be determined from the coordinates $(X'_{112a}, Y'_{112a})$ and $(X'_{112b}, Y'_{112b})$ of the magnetic source 112a, 112b, and this determined distance may be compared to the fixed distance d established between the magnetic sources 112a, 112b when the input device 100 is constructed. Variation between the determined distance and the fixed distance d may be indicative of an error, deformation, or other malfunction of the input device 100. The method may further comprise communicating an error via the control unit 300 to a user when the determined distance is different from the fixed distance d.

FIG. 9 illustrates, schematically, a computer-assisted surgery system 200 being represented in an operating room 201. The computer-assisted surgery system 200 comprises at least one robotic arm 210 extending between a first end 211 and a second end 212. The first end 211 may be attached to a base 220 of the computer-assisted system 200. The second end 212 forms a flange of the robotic arm 210 to which an end-effector 215 is fixed. The end-effector 215 may also comprise part of the robotic arm 210 located before its flange, that is to say between the base 220 and the flange.

The base 220 may be a movable base and comprises a mechanism, such as wheels 221, permitting movement of the base 220 within the operating room 201. By way of example, in a first operating mode, movement of the base 220 may be done using the input device 100. Accordingly, the user of the system 200 is able to easily move the base 220 depending on the treatment to be performed. In other non-illustrated embodiments, the base may be movable along rails or it may alternately be a fixed base, or it may be arranged on any suitable device. The base 220 further comprising mechanisms configured to lock the position of such base during the planned treatment.

At least three motorized joints 213 are formed between the first end 211 and the second end 212 of the robotic arm 210. More than three motorized joints 213 may be formed between the first end 211 and the second end 212 of the robotic arm 210. As used herein, the term "motorized joint" refers to a joint driven by its own motor, such joint being configured to be subjected to a linear deformation or to an angular deformation, within the scope of the invention. In other words, if n joints are formed between the first end 211 and the second end 212 of the robotic arm 210, the robotic arm 210 comprises n motors, each of which being configured to drive one of the j oints.

The robotic arm 210 comprises several segments 218. As illustrated in FIG. 9, each segment 218 is separated from the next one by at least one motorized joint 213. As used herein, the term "end-effector" designated as element 215 refers to the last segment 218 of the robotic arm 210, that is to say the segment of such robotic arm 210 positioned the farthest from the base 220 of the system 210, or the second to last segment 218 of the robotic arm 210. In some non-illustrated embodiments, the end-effector 215 may comprise one or several motorized joint(s) and the end-effector 215 may thus comprise at least two segments 218 of the robotic arm 210. The end-effector 215 may itself encompass one or several additional segments and related degrees of freedom, such as a power tool in translation, irrigation and suction apparatus, safety observation instrumentation, etc.

The robotic arm 210 illustrated in FIG. 9 is a serial robotic arm, but this robotic arm could be a parallel robotic arm, or a combination thereof within the scope of the invention. In some embodiments, the robotic arm may present at least six motorized joints.

The computer-assisted surgery system 200 also comprises at least one surgical tool 230. The surgical tool 230 is configured to be used to perform a treatment on an anatomical structure 250. The system 200 also comprises the input device 100. By way of example, in a second operating mode, movement of the robotic arm 210 and/or of the surgical tool 230 may be done using the input device 100. The surgical tool 230 and the input device 100 may both be attached to the robotic arm 210. The input device 100 and the surgical tool 230 may be considered to form part of the end-effector 215 of the robotic arm 210. As the surgical tool 230 and the input device 100 form part of the end-effector 215, it is understood, from what have been described above referring to the end-effector, that such surgical tool 230 and such input device 100 may be arranged on the last segment 218 of the robotic arm 210 or on the second to last segment 218 of such robotic arm 210. According to the illustrated embodiment, the surgical tool 230 and the input device 100 are fixed to the flange of the robotic arm 210. For instance, the surgical tool 230 may be fixed to such flange thanks to a shaft 114. Alternately, the surgical tool 230 may be directly mounted on the flange of the robotic arm 210. According to a non-illustrated embodiment, the input device 100 may be directly fixed to the surgical tool 230.

By way of example and not limitation, the surgical tool 230 may comprise at least one power tool configured to drive a tool. The tool may for instance be a cutting tool, such as a saw, a drill, a reamer or a burr. Alternatively, the surgical tool 230 may be a cutting guide or an insertion guide. Other known surgical tool 230 may be used without departing from the scope of the present invention depending upon the procedure to be performed on a given anatomical structure. Any surgical tool that is configured to act on or to treat an anatomical structure may be attached to the robotic arm within the scope of the invention. By way of further non-limiting examples, the surgical tool may be also an ultrasonic bone scalpel, a bone shaver, a laser that may cuts tissues or bones, a knife, a lancet, a cryosurgery probe, any radiofrequency tool, a microwave probe, a waterjet device, or a screwdriver.

By way of example and not limitation, the anatomical structure 250 may be a bone of a patient. According to the illustrated embodiment, the anatomical structure 250 is for example a tibial bone of the patient. As previously mentioned, the illustrated embodiment is only one example of how to carry the invention and the anatomical structure could be any other anatomical structure of said patient within the scope of the invention. For instance, this anatomical structure could be a femur bone, a shoulder scapula or humerus, a maxillo-facial bone, a small hand or foot bone such as metatarsal bone or talus, a vertebra, a pelvis, a tooth or mandible, a skull, a brain, and so forth. The system 200 of the disclosure is particularly well suited to be used during orthopedic, ENT, cranio-facial, dental surgeries, or neurosurgery.

The system 200 further comprises at least one control unit 300. According to the illustrated embodiment, the control unit 300 is integrated within the base 220 but such control unit 300 could be remote from the base 220 without departing from the scope of the invention. Further, while the control unit 300 is described with reference to a CAS system 200, the control unit 300 may be used in association with another system such as a gaming system in which the input device 100 is utilized. In another system, such as the gaming system, the control unit 300 or one or more components thereof may be located within or otherwise considered part of the input device 100 rather than being remote from the input device 100 as shown in FIG. 9.

The control unit 300 is configured to perform one or more of the processes described above. The control unit 300 may comprise a processor 302, a memory 304, and a storage device 306, which may be communicatively coupled by way of a communication infrastructure 308 as illustrated in FIG. 10.

While an example control unit 300 is shown in FIG. 10, the components illustrated in FIG. 9 are not intended to be limiting. Additional or alternative components may be used in other embodiments. Furthermore, in certain embodiments, the control unit 300 can include fewer components than those shown in FIG. 10.

The processor 302 includes hardware for executing instructions, such as those making up a computer program. By way of non-limiting example, to executive instructions, the processor 302 may retrieve (or fetch) the instructions from an internal register, an internal cache, the memory 304, or the storage device 322 and decode and execute them. By way of non-limiting example, the control unit 300 may include one or more instruction caches, one or more data caches, and one or more translation look aside buffers (TLBs). Instructions in the instruction caches may be copies of instructions in the memory 304 or the storage device 306. The processor 302 is communicatively coupled to the memory 304.

The memory 304 may be used for storing data, metadata, and programs for execution by the processor(s). The memory 304 may include one or more of volatile and nonvolatile memories, such as Random Access Memory ("RAM"), Read Only Memory ("ROM"), a solid state disk ("SSD"), Flash, Phase Change Memory ("PCM"), or other types of data storage. The memory 304 may be internal or distributed memory. As previously described herein, the memory 304 may store information related to the position and arrangements of the magnetic sources 112, sensors 114, and input device 100.

The control unit 300 is configured to instruct movement(s) to the motorized joints 213 of the robotic arm 210 which holds the surgical tool 230. The instructions configured to be sent by the control unit 300 are computed by the processor 302 based on the displacements of the input device 100, which results in displacement of the movable part 104 and the magnetic source(s) 112 as described herein.

The control unit 300 may be a computing device. The computing device may comprise or utilize a special purpose or general-purpose computer including computer hardware, such as, for example, one or more processors and system memory, as discussed in greater detail below. Embodiments within the scope of the present disclosure also include physical and other computer-readable media for carrying or storing computer-executable instructions and/or data structures. In particular, one or more of the processes described herein may be implemented at least in part as instructions embodied in a non-transitory computer-readable medium and executable by one or more computing devices (e.g., any of the media content access devices described herein). In general, a processor (e.g., a microprocessor) receives instructions, from a non-transitory computer-readable medium (e.g., a memory, etc.), and executes those instructions, thereby performing one or more processes, including one or more of the processes described herein.

Computer-readable media can be any available media that can be accessed by a general purpose or special purpose computer system. Computer-readable media that store computer-executable instructions are non-transitory computer-readable storage media (devices). Computer-readable media that carry computer-executable instructions are transmission media. Thus, by way of example, and not limitation, embodiments of the disclosure can comprise at least two distinctly different kinds of computer-readable media: non-transitory computer-readable storage media (devices) and transmission media.

Non-transitory computer-readable storage media (devices) includes RAM, ROM, EEPROM, CD-ROM, solid state drives ("SSDs") (e.g., based on RAM), Flash memory, phase-change memory ("PCM"), other types of memory, other optical disk storage, magnetic disk storage or other magnetic storage devices, or any other medium which can be used to store desired program code means in the form of computer-executable instructions or data structures and which can be accessed by a general purpose or special purpose computer.

Further, upon reaching various computer system components, program code means in the form of computer-executable instructions or data structures can be transferred automatically from transmission media to non-transitory computer-readable storage media (devices) (or vice versa). For example, computer-executable instructions or data structures received over a network or data link can be buffered in RAM within a network interface module (e.g., a "MC"), and then eventually transferred to computer system RAM and/or to less volatile computer storage media (devices) at a computer system. Thus, it should be understood that non-transitory computer-readable storage media (devices) can be included in computer system components that also (or even primarily) utilize transmission media.

Computer-executable instructions comprise, for example, instructions and data which, when executed at a processor, cause a general-purpose computer, special purpose computer, or special purpose processing device to perform a certain function or group of functions. In some embodiments, computer-executable instructions are executed on a general-purpose computer to turn the general-purpose computer into a special purpose computer implementing elements of the disclosure. The computer executable instructions may be, for example, binaries, intermediate format instructions such as assembly language, or even source code. Although the subject matter has been described in language specific to structural features and/or methodological acts, it is to be understood that the subject matter defined in the appended claims is not necessarily limited to the described features or acts described above. Rather, the described features and acts are disclosed as example forms of implementing the claims.

Those skilled in the art will appreciate that the disclosure may be practiced in network computing environments with many types of computer system configurations, including, personal computers, desktop computers, laptop computers, message processors, hand-held devices, multi-processor systems, microprocessor-based or programmable consumer electronics, network PCs, minicomputers, mainframe computers, mobile telephones, PDAs, tablets, pagers, routers, switches, and the like. The disclosure may also be practiced in distributed system environments where local and remote computer systems, which are linked (either by hardwired data links, wireless data links, or by a combination of hardwired and wireless data links) through a network, both perform tasks. In a distributed system environment, program modules may be located in both local and remote memory storage devices.

While the present disclosure has been described herein with respect to certain illustrated embodiments, those of ordinary skill in the art will recognize and appreciate that it is not so limited. Rather, many additions, deletions, and modifications to the illustrated embodiments may be made without departing from the scope of the disclosure, including legal equivalents thereof. In addition, features from one embodiment may be combined with features of another embodiment while still being encompassed within the scope of the invention as contemplated by the inventors.

## Claims

1.  A method for determining a position of a movable part (102) of an input device (100) relative to a static part (104) of the input device (100), the method comprising:

    measuring a magnetic field generated by a magnetic source (112) with a sensor array (116), the sensor array (116) comprising a plurality of unidirectional sensors (114) that measure a portion of the magnetic field parallel to a direction of magnetization of the magnetic source (112), the magnetic source (112) coupled to one of the movable part (102) and the static part (104) and the sensor array (116) coupled to the other of the movable part (102) and the static part (104), the movable part (102) is displaceable relative to the static part (104) according to at least two and at most three degrees of freedom, the degrees of freedom being two degrees of translational displacement and one degree of rotational freedom; and
    determining a location within a coordinate system (122) of the sensor array (116) at which the magnitude of the magnetic field of the magnetic source (112) is the greatest.

2.  The method of claim 1, further comprising determining a displacement of the magnetic source (112) based on the location at which the magnitude of the magnetic field of the magnetic source (112) is the greatest relative to an initial position of the magnetic source (112) within the coordinate system (122) of the sensor array (116).

3.  The method of any of claim 1 or 2, wherein determining a location at which the magnitude of the magnetic field of the magnetic source (112) is the greatest comprises:

    determining a surface (126) fit to the plurality of discrete magnetic field measurements of the sensors (114) and determining an apex (128) of the surface (126) and a location of the apex (128) within the coordinate system of the sensor array (116); or
    computing a centroid of the plurality of discrete magnetic field measurements of the sensors (114).

4.  The method of any of claims 1-3, wherein determining the displacement of the magnetic source (112) comprises determining at least one of translational displacement and angular displacement of the magnetic source (112) relative to an initial position.

5.  The method of any of claims 1-4, wherein measuring the magnetic field generated by the magnetic source (112) with the sensor array (116) comprises:

    measuring a magnetic field generated by a first magnetic source (112a) with a first portion of the sensor array (116); and
    measuring a magnetic field generated by a second magnetic source (112b) with a second portion of the sensor array (116),

wherein the first magnetic source (112a) and the second magnetic source (112b) are coupled to the one of the movable part (102) and the static part (104) such that the first and second magnetic sources (112a, 112b) are separated by a fixed distance (d).

6. The method of claim 5, wherein determining the displacement of the magnetic source (112) relative to the initial position of the magnetic source (112) comprises determining a rotational displacement of the first magnetic source (112a) and the second magnetic source (112b).

7. The method of any of claims 1-6, further comprising displacing a part of a system to which the input device (100) is operatively connected based upon the determined displacement of the magnetic source (112).

8. The method of any of claims 1-7, further comprising, prior to determining the location at which the magnitude of the magnetic field of the magnetic source (112) is the greatest, detecting an outlier magnetic field measurement from the measured magnetic field of the sensors (114) of the sensor array (116).

9. The method of claim 8, further comprising removing the outlier magnetic field measurement and using remaining magnetic field measurements to determine the location at which the magnitude of the magnetic field of the magnetic source (112) is the greatest.

10. The method of claim 8, further comprising communicating an error to a user based on the outlier magnetic field measurements.

11. A system (110) for tracking displacement of a movable part (102) of an input device (100) relative to a static part (104) of the input device (100), comprising:

at least one magnetic source (112) coupled to one of the movable part (102) and the static part (104), wherein the movable part (102) exhibits three degrees of freedom, the three degrees of freedom consisting of two degrees of translational freedom and one degree of rotational freedom;
at least one array of sensors (114) coupled to the other of the movable part (102) and the static part (104), and
a control unit (300) configured to perform the method of any of claims 1-10.

12. The system (110) of claim 11, wherein the sensors (114) are Hall effect sensors.

13. The system (110) of claim 11 or 12, wherein the at least one magnetic source (112) is a permanent magnet.

14. The system (110) of any of claims 11-13, wherein the at least one magnetic source (112) is coupled to the one of the movable part (102) and the static part (104) such that a direction of magnetization of the at least one magnetic source is parallel to an axis of rotational freedom of the input device (100).

15. A computer-assisted surgical system (200) comprising:

a robotic arm (210) with a plurality of motorized joints (213);
a surgical tool (230) coupled to the robotic arm (210); and

the system according to any of claims 11-14, wherein the control unit (300) is configured to displace at least one of the robotic arm (210) and the surgical tool (230) based on the determined displacement of the magnetic source (112).

112

d

112

Y

X

124

102

**FIGURE 1**

116

Y

114

X

122

104

**FIGURE 2**

113

112

110

118

Z

116

115

X, Y

$S_1$ $S_2$ $S_3$ $S_4$ $S_5$ $S_6$ $S_7$

114

**FIGURE 3**

**FIGURE 4**

**FIGURE 5**

FIGURE 6

FIGURE 7

14

**FIGURE 8a**

**FIGURE 8b**

**FIGURE 9**

**FIGURE 10**

Europäisches
Patentamt

European
Patent Office

Office européen
des brevets

## EUROPEAN SEARCH REPORT

Application Number

EP 23 30 5551

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 10 635 172 B1 (KELLER SEAN JASON [US] ET AL) 28 April 2020 (2020-04-28) | 1-14 | INV. G06F3/0346 |
| Y | * column 4 - column 7; figures 1-6 * | 15 | |
| Y,D | WO 2022/018691 A1 (UNIV DEGLI STUDI DI SIENA [IT]) 27 January 2022 (2022-01-27) * paragraphs [0076], [0077] * | 15 | |

TECHNICAL FIELDS
SEARCHED        (IPC)

G06F

The present search report has been drawn up for all claims

| Place of search | Date of completion of the search | Examiner |
|---|---|---|
| Munich | 27 September 2023 | Benzina, Amal |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
...........................................................................................
& : member of the same patent family, corresponding
document

EPO FORM 1503 03.82 (P04C01)

**ANNEX TO THE EUROPEAN SEARCH REPORT**
**ON EUROPEAN PATENT APPLICATION NO.**

EP 23 30 5551

This annex lists the patent family members relating to the patent documents cited in the above-mentioned European search report.
The members are as contained in the European Patent Office EDP file on
The European Patent Office is in no way liable for these particulars which are merely given for the purpose of information.

27-09-2023

| Patent document cited in search report | | Publication date | Patent family member(s) | | Publication date |
|---|---|---|---|---|---|
| US 10635172 | B1 | 28-04-2020 | NONE | | |
| WO 2022018691 | A1 | 27-01-2022 | EP | 4185941 A1 | 31-05-2023 |
| | | | WO | 2022018691 A1 | 27-01-2022 |

**REFERENCES CITED IN THE DESCRIPTION**

**Patent documents cited in the description**

- US 8862200 B
- WO 2022018691 A

- US 886220 A